# EUROPÄISCHE PATENTANMELDUNG

(11) **EP 3 616 656 A1**
(43) Veröffentlichungstag der Anmeldung: **04.03.2020**
(21) Anmeldenummer: 18191432.6
(22) Anmeldetag: 29.08.2018
(51) Int. Cl.: A61F 2/915, A61L 31/18

(54) **RÖNTGENMARKER FÜR EINE GEFÄSSSTÜTZE**

(71) Anmelder: BIOTRONIK AG, 8180 Bülach (CH)
(72) Erfinder: Fargahi, Amir, 8180 Bülach (CH)
(74) Vertreter: Galander, Marcus

(57) **Zusammenfassung**

Die Erfindung betrifft eine Gefäßstütze (1), wobei die Gefäßstütze (1) einen Gefäßstützenkörper (2) mit einer Mehrzahl an miteinander verbundenen Streben (20) aufweist, und wobei die Gefäßstütze (1) einen Röntgenmarker (3) aufweist. Erfindungsgemäß ist vorgesehen, dass der Röntgenmarker (3) an einem flexiblen Trägerelement (4) festgelegt ist, wobei das Trägerelement (4) zumindest einen Abschnitt (21) des Gefäßstützenkörpers (2) umgreift, so dass der Röntgenmarker (3) über das Trägerelement (4) an dem Gefäßstützenkörper (2) aufgehängt ist. Weiterhin betrifft die Erfindung ein Verfahren zur Herstellung einer Gefäßstütze (1).

## Beschreibung

Die Erfindung betrifft ein Implantat mit einem Grundkörper der mindestens eine Strebe aufweist. Die vorliegende Erfindung eignet sich insbesondere für eine Gefäßstütze, insbesondere eine biodegradierbare Gefäßstütze, die einen Gefäßstützenkörper aufweist, der z. B. aus einer Magnesiumlegierung gebildet sein kann, und wird anhand dieser Ausführungsform beschrieben. Die Erfindung ist jedoch prinzipiell nicht darauf limitiert sondern für jedes Implantat mit mindestens einer Strebe geeignet.

Derartige Gefäßstützen werden z. B. verwendet, um ein verengtes Gefäß eines Patienten offenzuhalten bzw. die umgebende Gefäßwand zu stützen und können z. B. mittels eines Ballonkatheters in bekannter Weise implantiert werden.

Die für eine solche Gefäßstütze verwendeten Materialien wie z. B. Mg, Nitinol, Co-Cr und Stahllegierungen sind nicht immer genügend röntgendicht. Um die betreffende Gefäßstütze daher im Röntgenbild sichtbar zu machen, wird zumindest ein Röntgenmarker an der Gefäßstütze vorgesehen, der z. B. aus Ta, Pt, Au oder W bestehen kann.

Hinsichtlich der Röntgenmarker ergibt sich insbesondere im Zusammenhang mit metallischen Gefäßstützenkörpern das Risiko der Kontaktkorrosion, wenn der Röntgenmarker und der Gefäßstützenkörper in Kontakt geraten. Weiterhin sind oftmals Klebstoffe zur Verbindung des Röntgenmarkers mit dem Gefäßstützenkörper bedenklich im Hinblick auf die Biokompatibilität des Implantats. Weiterhin können starre Verbindungen zwischen dem Röntgenmarker und dem Gefäßstützenkörper zu einem Verlust des Markers beim Crimpen der Gefäßstütze führen.

Aus der US 2016/0228270 ist ein Röntgenmarker bekannt, der auf einem Polymersteg des Stents sitzt und diesen dabei umgreift.
Hiervon ausgehend liegt der vorliegenden Erfindung die Aufgabe zugrunde, eine verbesserte Gefäßstütze bereitzustellen, bei der insbesondere die Gefahr der oben genannten Kotaktkorrosion vermieden wird.

Diese Aufgabe wird durch ein Implantat mit den Merkmalen des Anspruchs 1 gelöst. Vorteilhafte Ausgestaltungen der Erfindung sind in den Unteransprüchen angegeben und werden nachfolgen detailliert beschrieben.

Gemäß Anspruch 1 wird ein Implantat offenbart, wobei das Implantat (1) einen Implantatkörper (2) mit mindestens einer Streben (20) aufweist, und wobei das Implantat (1) einen Röntgenmarker (3) aufweist.

Erfindungsgemäß ist nun vorgesehen, dass der Röntgenmarker an einem flexiblen Trägerelement festgelegt ist, wobei das Trägerelement zumindest einen Abschnitt des Implantatkörpers umgreift, so dass der Röntgenmarker über das Trägerelement an dem Implantatkörper aufgehängt ist.

Bevorzugt ist das Implantat als Gefäßstütze ausgeführt ist, wobei die Gefäßstütze einen Gefäßstützenkörper mit einer Mehrzahl an miteinander verbundenen Streben aufweist und wobei das Trägerelement bevorzugt eine Strebe umgreift. Eine derartige Gefäßstütze kann in ein Körpergefäß, wie beispielsweise ein Blutgefäß, implantiert werden und diese so stützen. Bei einem Blutgefäß dienen die Gefäßstützen häufig dazu, das Gefäß offen zu halten und werden zumeist als Stent bezeichnet.

Der Röntgenmarker ist vorzugsweise dazu ausgebildet, in einem Röntgenbild einen deutlich erkennbaren Kontrast zu bilden. Der Röntgenmarker besteht daher insbesondere aus einem entsprechend röntgenopaken Material. Gemäß einer Ausführungsform der Erfindung kann der Röntgenmarker, der vorzugsweise ein Solid-Röntgenmarker bzw. ein monolithischer Röntgenmarker ist, aus einem der folgenden Stoffe bestehen bzw. einen der folgenden Stoffe aufweisen: Pt, Au, W, Ta.

Bei dem Implantat handelt es sich vorzugsweise um ein biodegradierbares Implantat. D. h., das implantierte Implantat ist dazu ausgebildet, sich im Körper des Patienten innerhalb eines insbesondere definierten Zeitraums zu zersetzen.

Der Implantatkörper ist des Weiteren vorzugsweise metallisch ausgebildet. Der Implantatkörper kann jedoch auch nichtmetallische Bestandteile (z. B. in Form einer Beschichtung) aufweisen.

Gemäß einer Ausführungsform der Erfindung ist vorgesehen, dass der Implantatkörper aus einem der folgenden Stoffe gebildet ist oder einen der folgenden Stoffe aufweist: ein Metall, Magnesium, eine Metalllegierung, eine Magnesiumlegierung. Vorteilhafterweise ist der Implantatkörper aus einer Magnesiumlegierung enthaltend seltene Erden (Y, Nd, Zr, Gd und/oder Dy) als Legierungselemente oder aus einer Magnesiumlegierung enthaltend Zn, Ca und/oder Al als Legierungselement (beispielsweise MgAl, MgZnAl oder MgZnCa).

Die Magnesiumlegierung ist insbesondere gemäß einer Ausführungsform so gewählt, dass der Grundkörper (insbesondere über einen definierten Zeitraum hinweg) biodegradierbar bzw. im Körper des Patienten zersetzbar ist.

Gemäß einer Ausführungsform der Erfindung ist vorgesehen, dass die Implantat von einem Ausgangszustand, in dem der Implantatkörper in einer radialen Richtung des Implantatkörpers komprimiert ist bzw. gecrimpt ist, in einen expandierten (oder dilatierten) Zustand überführbar ist, in dem die Implantat in radialer Richtung expandiert ist (z. B. mittels eines Ballonkatheters). Die jeweilige radiale Richtung steht dabei senkrecht auf der axialen Richtung des Implantatkörpers.

Weiterhin ist gemäß einer Ausführungsform des erfindungsgemäßen Implantats vorgesehen, dass der Röntgenmarker über das Trägerelement so an dem Implantatkörper aufgehängt ist, dass er beabstandet zum Implantatkörper angeordnet ist, d. h., der Röntgenmarker weist insbesondere keinerlei Kontakt zum Implantatkörper auf.

Weiterhin ist gemäß einer Ausführungsform des erfindungsgemäßen Implantats vorgesehen, dass das Trägerelement aus einem der folgenden Materialien besteht oder eines der folgenden Materialien aufweist: ein Kunststoff, ein Polymer, ein Polylactid (PLA).

Weiterhin ist gemäß einer Ausführungsform des erfindungsgemäßen Implantats vorgesehen, dass sich der Implantatkörper in einer axialen Richtung erstreckt, wobei sich die Streben jeweils in einer Umfangsrichtung des Implantatkörpers erstrecken (wobei die jeweilige Strebe insbesondere in der Umfangsrichtung umläuft, d. h., ringförmig ausgebildet ist).

Weiterhin ist gemäß einer Ausführungsform des erfindungsgemäßen Implantats vorgesehen, dass die jeweilige Strebe eine Mehrzahl an miteinander verbundenen gekrümmten Abschnitten (insbesondere in Form von bogenförmigen Abschnitten) aufweist, wobei die gekrümmten Abschnitte bezogen auf die besagte axiale Richtung alternierend angeordnete Minima und Maxima bilden Mit anderen Worten weist also die jeweilige Strebe einen mäanderförmigen Verlauf auf.

Weiterhin ist gemäß einer Ausführungsform des erfindungsgemäßen Implantats vorgesehen, dass zwei in axialer Richtung benachbarte Streben über zumindest einen Steg miteinander verbunden sind. Vorzugsweise wird eine solche Verbindung über zwei oder drei solche Stege hergestellt.

Weiterhin ist gemäß einer Ausführungsform des erfindungsgemäßen Implantats vorgesehen, dass das Trägerelement einen ersten Abschnitt aufweist, der eine der Streben des Implantatkörpers umgreift, so dass das Trägerelement an dieser Strebe aufgehängt ist, und wobei das Trägerelement einen mit dem ersten Abschnitt des Trägerelements verbundenen freien zweiten Abschnitt aufweist, an dem der Röntgenmarker festgelegt ist.

Weiterhin ist gemäß einer alternativen Ausführungsform des erfindungsgemäßen Implantats vorgesehen, dass das Trägerelement einen ersten Abschnitt aufweist, der den mindestens einen Steg umgreift, so dass das Trägerelement an diesem Steg aufgehängt ist, und wobei das Trägerelement einen mit dem ersten Abschnitt des Trägerelements verbundenen freien zweiten Abschnitt aufweist, an dem der Röntgenmarker festgelegt ist.

Weiterhin ist gemäß einer Ausführungsform des erfindungsgemäßen Implantats vorgesehen, dass das Trägerelement ringförmig, insbesondere schlauchförmig, ausgebildet ist. Das Trägerelement kann insbesondere aus einer flachen, bandförmigen Materiallage gebildet sein, die in einer Ringform angeordnet bzw. zu einer Ringform verbunden wird. Das Trägerelement kann auch in Ringform extrudiert, geblasen oder spritzgegossen sein.

Das Trägerelement weist gemäß einer Ausführungsform eine Breite im Bereich von 0,5mm bis 2 mm auf. Weiterhin weist das Trägerelement gemäß einer Ausführungsform eine Wandstärke von z. B. 0,01 mm auf. Weiterhin weist das Trägerelement gemäß einer Ausführungsform einen Innendurchmesser im Bereich von 1,5 mm bis 2,5 mm auf.

Weiterhin ist gemäß einer Ausführungsform des erfindungsgemäßen Implantats vorgesehen, dass der besagte Abschnitt des Implantatkörpers, den das Trägerelement umgreift, einer der gekrümmten (bzw. bogenförmigen) Abschnitte ist (siehe oben).

Weiterhin ist gemäß einer Ausführungsform des erfindungsgemäßen Implantats vorgesehen, dass sich das Trägerelement zwischen zwei einander gegenüberliegenden Flanken dieses gekrümmten bzw. bogenförmigen Abschnitts erstreckt.

Weiterhin ist gemäß einer Ausführungsform des erfindungsgemäßen Implantats vorgesehen, dass der Röntgenmarker so an dem Trägerelement festgelegt ist, dass er zwischen den beiden Flanken des besagten gekrümmten (oder bogenförmigen) Abschnitts angeordnet ist.

Hierbei werden insbesondere auch zwei einander gegenüberliegende Abschnitte des ringförmigen Trägerelements mittels des Röntgenmarkers miteinander verbunden, so dass das Trägerelement zusammen mit dem Röntgenmarker verliersicher auf dem gekrümmten bzw. bogenförmigen Abschnitt der betreffenden Strebe des Implantatkörpers angeordnet ist bzw. an diesem aufgehängt ist.

Weiterhin ist gemäß einer Ausführungsform des erfindungsgemäßen Implantats vorgesehen, dass der Röntgenmarker als ein Niet oder als ein Bestandteil eines Niets ausgebildet ist, wobei der Niet mit dem Trägerelement vernietet ist, um den Röntgenmarker am Trägerelement festzulegen.

Der Niet kann also einteilig ausgebildet sein, kann jedoch auch aus mehreren Bestandteilen bestehen. Für den Fall, dass der Niet mehrteilig ausgebildet ist, kann ggf. nur einer der Bestandteile aus einem z. B. oben genannten röntgenopaken Material bestehen bzw. den Röntgenmarker bilden.

Ein weiterer Aspekt der vorliegenden Erfindung betrifft ein Verfahren zum Herstellen eines Implantat (insbesondere einer erfindungsgemäßen Gefäßstütze), wobei ein Implantatkörper mit einer Mehrzahl an miteinander verbundenen Streben, ein Röntgenmarker und ein flexibles Trägerelement bereitgestellt werden, wobei das Trägerelement so an dem Implantatkörper angeordnet wird, dass das Trägerelement einen Abschnitt des Implantatkörpers umgreift, und wobei der Röntgenmarker so an dem Trägerelement festgelegt wird, dass er beabstandet zum Implantatkörper angeordnet ist und über das Trägerelement an dem Abschnitt des Implantatkörpers aufgehängt ist.

Gemäß einer Ausführungsform des erfindungsgemäßen Verfahrens ist vorgesehen, dass sich der Implantatkörper in einer axialen Richtung erstreckt, wobei sich die Streben jeweils in einer Umfangsrichtung des Implantatkörpers erstrecken (wobei die jeweilige Strebe insbesondere in der Umfangsrichtung umläuft, d. h., ringförmig ausgebildet ist).

Weiterhin ist gemäß einer Ausführungsform des erfindungsgemäßen Verfahrens vorgesehen, dass die jeweilige Strebe eine Mehrzahl an miteinander verbundenen gekrümmten Abschnitten aufweist, wobei die gekrümmten Abschnitte bezogen auf die axiale Richtung alternierend angeordnete Minima und Maxima bilden (siehe auch oben).

Weiterhin ist gemäß einer Ausführungsform des erfindungsgemäßen Verfahrens vorgesehen, dass zwei in axialer Richtung benachbarte Streben über zumindest einen Steg miteinander verbunden sind.

Weiterhin ist gemäß einer Ausführungsform des erfindungsgemäßen Verfahrens vorgesehen, dass das Trägerelement mit einen ersten Abschnitt des Trägerelements so an einer der Streben oder an dem mindestens einen Steg aufgehängt wird, dass der erste Abschnitt die besagte Strebe bzw. den besagten Steg umgreift, wobei der Röntgenmarker an einem mit dem ersten Abschnitt des Trägerelements verbundenen freien zweiten Abschnitt des Trägerelements festgelegt wird. Das Trägerelement bildet hier also insbesondere einen von dem Implantatkörper abstehenden Flügel, der den Röntgenmarker trägt.

Weiterhin ist gemäß einer Ausführungsform des erfindungsgemäßen Implantats vorgesehen, dass der besagte Abschnitt, den das (vorzugswese ringförmige) Trägerelement umgreift einer der gekrümmten bzw. bogenförmigen Abschnitte ist (siehe auch oben), so dass sich insbesondere das Trägerelement zwischen zwei einander gegenüberliegenden Flanken des gekrümmten bzw. bogenförmigen Abschnitts erstreckt.

Weiterhin ist gemäß einer Ausführungsform des erfindungsgemäßen Implantats vorgesehen, dass der Röntgenmarker so an dem Trägerelement festgelegt wird, insbesondere mit diesem vernietet wird, dass er zwischen den beiden Flanken des besagten gekrümmten bzw. bogenförmigen Abschnitts angeordnet ist.

Das erfindungsgemäße Implantat, insbesondere die erfindungsgemäße Gefäßstütze, unterscheidet sich vom Stand der Technik vor allem dadurch, dass der Röntgenmarker selbst keinen direkten Kontakt mit dem Implantatkörper hat und das Trägerelement flexibel ausgeführt werden kann. Durch die flexible Ausführung des Trägerelements kann bei einer Gefäßstütze ein sehr kleines Profil für das Kathetersystem mit der komprimierten Gefäßstütze bei der Implantation erreicht werden.

Weitere Merkmale und Ausführungsformen der vorliegenden Erfindung sollen nachfolgend anhand der Figuren erläutert werden. Es zeigen:
- Fig. 1: eine ausschnitthafte perspektivische Ansicht einer Ausführungsform eines erfindungsgemäßen Implantats ausgeführt als Gefäßstütze mit einem Röntgenmarker;
- Fig. 2: ein Detail der Figur 1;
- Fig. 3: eine ausschnitthafte Ansicht einer Strebe des Gefäßstützenkörpers, an der der Röntgenmarker festgelegt werden soll;
- Fig. 4: eine perspektivische Ansicht eines ringförmigen Trägerelements;
- Fig. 5: das Trägerelement gemäß Figur 4 nach einem Aufschieben auf einen gekrümmten Abschnitt einer Strebe des Gefäßstützenkörpers; und
- Fig. 6: den an das Trägerelement festgelegten Röntgenmarker.

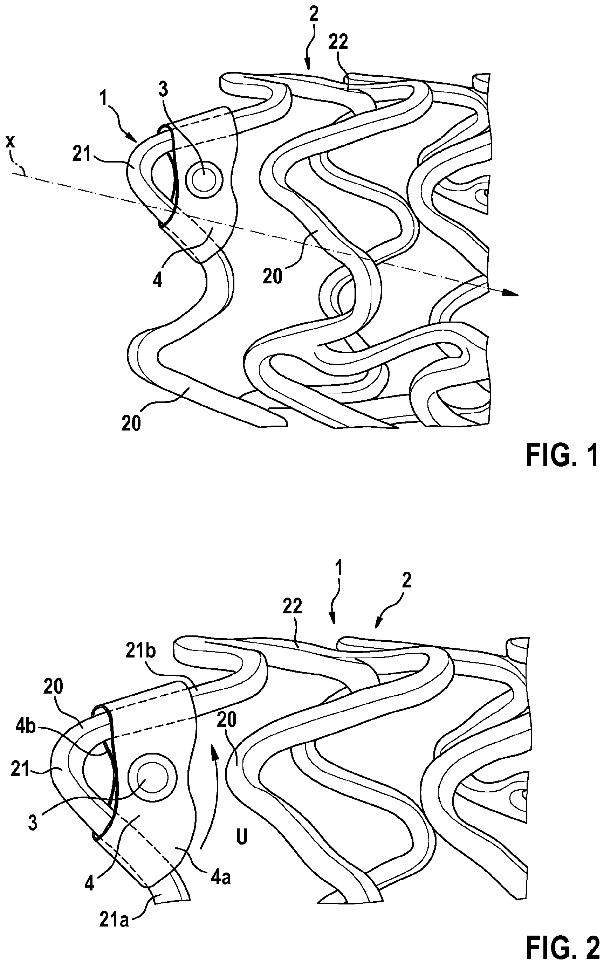

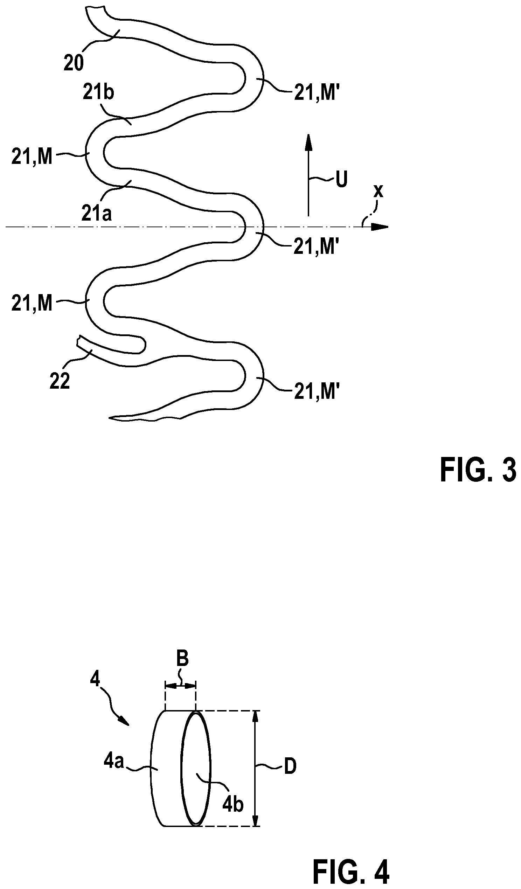

Figur 1 zeigt im Zusammenhang mit den Figuren 2 und 3 eine Ausführungsform einer erfindungsgemäßen Gefäßstütze 1, wobei die Gefäßstütze 1 einen Gefäßstützenkörper 2 mit einer Mehrzahl an miteinander verbundenen Streben 20 aufweist sowie einen Röntgenmarker 3 (z. B. aus Pt, Au, W, Ta). Erfindungsgemäß ist vorgesehen, dass der Röntgenmarker 3 an einem flexiblen Trägerelement 4 festgelegt ist, wobei das Trägerelement 4 zumindest einen Abschnitt 21 des Gefäßstützenkörpers 2 umgreift, so dass der Röntgenmarker 3 über das Trägerelement 4 an dem Gefäßstützenkörper 2 aufgehängt ist und dabei insbesondere beabstandet zum Gefäßstützenkörper 2 angeordnet ist. Hierbei besteht das Trägerelement 4 z. B. aus PLA. Der Gefäßstützenkörper 2 ist insbesondere metallisch und kann biodegradierbar ausgebildet sein. Hierfür kann der Gefäßstützenkörper aus einer geeigneten Magnesiumlegierung (oder einem anderen geeigneten Material) gebildet sein.

Wie den Figuren 1 und 2 zu entnehmen ist erstreckt sich der Gefäßstützenkörper 2 in einer axialen Richtung x, wobei sich die Streben 20 jeweils in einer Umfangsrichtung U des Gefäßstützenkörpers 2 erstrecken bzw. in der Umfangsrichtung U ringförmig umlaufen.

Dabei weist die jeweilige Strebe 20, wie insbesondere anhand der Figur 3 ersichtlich ist, 20 eine Mehrzahl an miteinander verbundenen gekrümmten Abschnitten 21 auf, wobei die gekrümmten Abschnitte 21 bezogen auf die axiale Richtung x alternierend angeordnete Minima M und Maxima M' bilden. Je zwei in axialer Richtung x benachbarte Streben 20 können über zumindest einen Steg 22 miteinander verbunden sein, so dass sich der gesamte Gefäßstützenkörper 2 ergibt.

Wie in der Figur 4 gezeigt ist, ist das Trägerelement 4 gemäß einer Ausführungsform ringförmig ausgebildet, und weist dabei zwei miteinander verbundene Abschnitte 4a, 4b auf. Das Trägerelement 4 kann weiterhin eine Breite B im Bereich von 0,5mm bis 2 mm und/oder einen Innendurchmesser D im Bereich von 1,5 mm bis 2,5 mm aufweisen. Die Wandstärke kann z. B. 0,01 mm betragen.

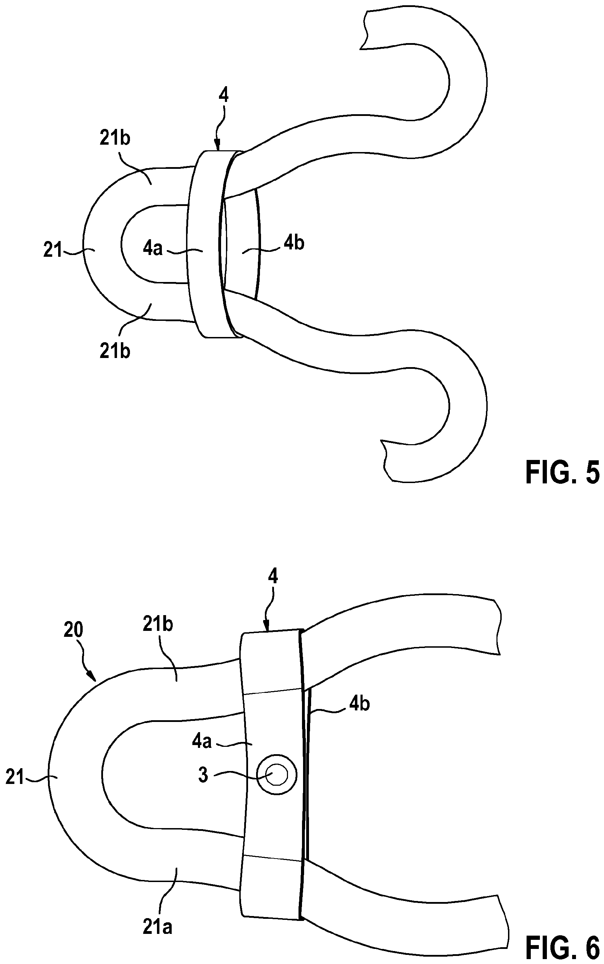

Zum Festlegen des Trägerelements 4 an dem Gefäßstützenkörper kann diese z. B. gemäß Figur 5 auf einen der gekrümmten Abschnitte 21 einer Strebe 20 des Gefäßstützenkörpers 2 aufgeschoben werden, so dass das Trägerelement 4 den Abschnitt 21 umgreift. Hierbei erstreckt sich das Trägerelement 4 zwischen zwei einander gegenüberliegenden Flanken 21a, 21b des gekrümmten Abschnitts 21 und erlaubt somit eine Aufhängung des Röntgenmarkers 3 an dem Abschnitt 21 bzw. an der entsprechenden Strebe 20 des Gefäßstützenkörpers 2. Hierzu wird der vorzugsweise monolithisch ausgestaltete Röntgenmarker 3 an dem Trägerelement 4 festgelegt, und zwar insbesondere so, dass die beiden Abschnitte 4a, 4b des Trägerelements 4 die auf beiden Seiten des Abschnitts 21 liegen ebenfalls miteinander verbunden werden, wie es in der Figur 6 gezeigt ist. Dies erlaubt insbesondere eine verliersichere Anordnung des Röntgenmarkers 3 an dem Gefäßstützenkörper 2.

Gemäß einer Ausführungsform kann der Röntgenmarker 3 als ein Niet oder als ein Bestandteil eines Niets ausgebildet sein. Beim Vernieten des Niets 3 bzw. Röntgenmarkers 3 mit dem Trägerelement 4 kann sogleich die besagte Verbindung zwischen den beiden Abschnitten 4a, 4b des Trägerelements über den Niet/Röntgenmarker 3 hergestellt werden.

## Patentansprüche

1. Implantat (1), wobei das Implantat (1) einen Implantatkörper (2) mit mindestens einer Streben (20) aufweist, und wobei das Implantat (1) einen Röntgenmarker (3) aufweist,
**dadurch gekennzeichnet,**
**dass** der Röntgenmarker (3) an einem flexiblen Trägerelement (4) festgelegt ist, wobei das Trägerelement (4) zumindest einen Abschnitt (21) des Implantatkörpers (2) umgreift, so dass der Röntgenmarker (3) über das Trägerelement (4) an dem Implantatkörper (2) aufgehängt ist.

2. Implantat nach Anspruch 1, **dadurch gekennzeichnet, dass** das Implantat als Gefäßstütze ausgeführt ist, wobei die Gefäßstütze einen Gefäßstützenkörper mit einer Mehrzahl an miteinander verbundenen Streben (20) aufweist und wobei das Trägerelement (4) bevorzugt eine Strebe umgreift.

3. Implantat nach Anspruch 1 oder 2, **dadurch gekennzeichnet, dass** der Röntgenmarker (3) über das Trägerelement (4) so an dem Implantatkörper (2) aufgehängt ist, dass er beabstandet zum Implantatkörper (2) angeordnet ist.

4. Implantat nach einem der vorhergehenden Ansprüche, **dadurch gekennzeichnet, dass** das Trägerelement (4) aus einem der folgenden Materialien besteht oder eines der folgenden Materialien aufweist: ein Kunststoff, ein Polymer, ein Polylactid (PLA).

5. Implantat nach einem der Ansprüche 2 bis 4, **dadurch gekennzeichnet, dass** sich der Gefäßstützenkörper (2) in einer axialen Richtung (x) erstreckt, wobei sich die Streben (20) jeweils in einer Umfangsrichtung (U) des Gefäßstützenkörpers (2) erstrecken.

6. Implantat nach einem der Ansprüche 2 bis 5, **dadurch gekennzeichnet, dass** die jeweilige Strebe (20) eine Mehrzahl an miteinander verbundenen gekrümmten Abschnitten (21) aufweist, wobei die gekrümmten Abschnitte (21) bezogen auf die axiale Richtung (x) alternierend angeordnete Minima (M) und Maxima (M') bilden.

7. Implantat nach einem der Ansprüche 2 bis 6, **dadurch gekennzeichnet, dass** zwei in axialer Richtung (x) benachbarte Streben (20) über zumindest einen Steg (22) miteinander verbunden sind.

8. Implantat nach einem der vorhergehenden Ansprüche, **dadurch gekennzeichnet, dass** das Trägerelement einen ersten Abschnitt aufweist, der eine der Streben des Implantatkörpers umgreift, so dass das Trägerelement an dieser Strebe aufgehängt ist, und wobei das Trägerelement einen mit dem ersten Abschnitt des Trägerelements verbundenen freien zweiten Abschnitt aufweist, an dem der Röntgenmarker festgelegt ist.

9. Implantat nach Anspruch 7, **dadurch gekennzeichnet, dass** das Trägerelement einen ersten Abschnitt aufweist, der den mindestens einen Steg umgreift, so dass das Trägerelement an diesem Steg aufgehängt ist, und wobei das Trägerelement einen mit dem ersten Abschnitt des Trägerelements verbundenen freien zweiten Abschnitt aufweist, an dem der Röntgenmarker festgelegt ist.

10. Implantat nach einem der Ansprüche 1 bis 7, **dadurch gekennzeichnet, dass** das Trägerelement (4) ringförmig ausgebildet ist.

11. Implantat nach einem der Ansprüche 1 bis 7, 9, **dadurch gekennzeichnet, dass** der besagte Abschnitt (21), den das Trägerelement (4) umgreift einer der gekrümmten Abschnitte (21) ist.

12. Implantat nach Anspruch 11, **dadurch gekennzeichnet, dass** sich das Trägerelement (4) zwischen zwei einander gegenüberliegenden Flanken (21a, 21b) des gekrümmten Abschnitts (21) erstreckt.

13. Implantat nach Anspruch 12, **dadurch gekennzeichnet, dass** der Röntgenmarker (3) so an dem Trägerelement (4) festgelegt ist, dass er zwischen den beiden Flanken (21a, 21b) des besagten gekrümmten Abschnitts (21) angeordnet ist.

14. Implantat nach einem der vorhergehenden Ansprüche, **dadurch gekennzeichnet, dass** der Röntgenmarker (3) als ein Niet oder als ein Bestandteil eines Niets ausgebildet ist, wobei der Niet mit dem Trägerelement (4) vernietet ist.

15. Implantat nach einem der vorhergehenden Ansprüche, **dadurch gekennzeichnet, dass** der Gefäßstützenkörper (2) aus einem der folgenden Stoffe gebildet ist oder einen der folgenden Stoffe aufweist: ein Metall, Magnesium, eine Metalllegierung, eine Magnesiumlegierung.

16. Verfahren zum Herstellen eines Implantats, insbesondere einer Gefäßstütze (1), wobei ein Implantatkörper (2) mit einer Mehrzahl an miteinander verbundenen Streben (20), ein Röntgenmarker (3) und ein flexibles Trägerelement (4) bereitgestellt werden, wobei das Trägerelement (4) so an dem Implantatkörper (2) angeordnet wird, dass das Trägerelement (4) einen Abschnitt (21) des Implantatkörpers (2) umgreift, und wobei der Röntgenmarker (3) so an dem Trägerelement (4) festgelegt wird, dass er beabstandet zum Implantatkörper (2) angeordnet ist und über das Trägerelement (4) an dem Abschnitt (21) des Implantatkörpers (2) aufgehängt ist.
